# EUROPEAN PATENT SPECIFICATION

(11) **EP 2 424 979 B1**
(45) Date of publication and mention of the grant of the patent: **23.07.2014**
(21) Application number: 10727861.6
(22) Date of filing: 28.04.2010
(51) Int. Cl.: C12N 5/10, A61K 35/12, A61K 35/00, A61K 48/00

(54) **METHOD FOR PRODUCTION OF ANTI-TUMOR TRAIL PROTEIN**
VERFAHREN ZUR HERSTELLUNG VON ANTI-TUMORALES TRAIL PROTEIN
MÉTHODE DE PRÉPARATION DE LA PROTÉINE ANTI-TUMORALE TRAIL

(30) Priority: 28.04.2009 IT MO20090100
(43) Date of publication of application: 07.03.2012
(73) Proprietor: Dominici, Massimo, 44100 Ferrara (FE) (IT); Bussolari, Rita, 40014 Crevalcore (BO) (IT); Grisendi, Giulia, 42100 Reggio Emilia (RE) (IT); Conte, Pierfranco, 56100 Pisa (PI) (IT)
(72) Inventor: Dominici, Massimo, 44100 Ferrara (FE) (IT); Bussolari, Rita, 40014 Crevalcore (BO) (IT); Grisendi, Giulia, 42100 Reggio Emilia (RE) (IT); Conte, Pierfranco, 56100 Pisa (PI) (IT)
(74) Representative: Bergamini, Silvio
(86) International application number: PCT/IB2010/051850
(87) International publication number: WO 2010/125527

(56) References cited:
- SHI J. ET AL.: "Overexpression of soluble TRAIL induces apoptosis in human lung adenocarcinoma and inhibits growth of tumor xenograft in nude mice" CANCER RES., vol. 65, no. 5, 2005, pages 1687-1692, XP002563286
- MOHR A. ET AL: "Mesenchymal stem cells expressing TRAIL lead to tumour growth inhibition in an experimental lung cancer model" J. CELL. MOL. MED., vol. 12, no. 6b, 2008, pages 2628-2643, XP002563288
- COURTETE J. ET AL: [Online] 2008, INTERNATIONAL ADVANCED ICAS/APOPTRAIN TRAINING COURSE ON ADVANCES IN CELL DEATH RESEARCH , XP002563289 Retrieved from the Internet: URL:http://www.uniklinik-ulm.de/fileadmin/ Kliniken/ApopTrain/public/apoptrain2008.pd f> [retrieved on 2010-01-14] page 30
- ZHANG Y. ET AL: "pVax1 plasmid vector-mediated gene transfer of soluble TRAIL suppresses human hepatocellular carcinoma growth in nude mice" ACTA BIOCHIM POL, vol. 54, no. 2, 2007, pages 307-313, XP002563287

## Description

### Field of the invention

The present invention relates to an anti-tumor medicament that comprises adipose pericytes (AD-PC) stably expressing soluble TRAIL (sTRAIL) from a retroviral vector.

### Background art

The word TRAIL is known to technically define a molecule belonging to the family of the so-called "death ligands", i.e. a family of Tumor Necrosis Factor molecules (TNF).

In practice, a TRAIL molecule can induce cell death in diseased tissues only, and spare healthy tissues, and for this characteristic it is a particularly interesting molecule for use in oncological treatments and in other biomedical fields.

TRAIL-based treatment of an organism affected by tumor cells may occur in two particular manners: in the former, the TRAIL molecule is chemically synthesized beforehand, in which case it is defined as recombinant TRAIL, and later administered to the tumor-affected organism; in the latter the TRAIL molecule may be introduced into a tumor-affected organism through a carrier consisting of a TRAIL-producing cell.

In the former case, the need was found for combined use of chemotherapeutic agents to enhance anti-tumor effects, because the latter have been found to progressively decrease, due to the very short half-life of the TRAIL molecule, i.e. of the order of 20/30 minutes, the high renal excretion rate of this molecule, and TRAIL resistance. The combination with chemotherapeutic drugs encourages the use of the recombinant TRAIL molecule due to its specific tumor cell eliminating capacity, but this combination is affected by high toxicity toward hepatocytes, lymphocytes and osteoblasts, and leads to undesired side effects for the organism.

Furthermore, due to short half-life and high excretion rate, repeated administration of chemotherapeutic drugs was required in combination with TRAIL and this caused a considerable increase of overall costs upon repeated TRAIL administrations. In the latter case, the TRAIL molecule is contained in a virus that is used as a vector therefor and allows release of its genetic makeup containing the TRAIL-encoding sequence, which is later translated into a protein and transferred to the membrane of the cell infected by such viral vector.

These infected cells become the medium that carries TRAIL in direct contact with target tumor cells, thereby causing apoptosis thereof.

The viral vectors used heretofore are vectors that belong to the lentivirus or adenovirus family

The cells infected by these vectors can carry the TRAIL molecule to the location of the organism in which an anti-tumor effect, i.e. apoptosis of neoplastic cells is required.

For this purpose, multiple cell types are used, which all have a particular tropism for tumor disease sites: for example, hematopoietic stem cells and mesenchymal stem cells are used.

Prior art discloses human cells infected with a recombinant AAV2 (see : Shi, Cancer Res. 65(5): 1687-1692 (2005)) or a plasmamid (see : Zhang, Acta Biochim. Pol 54(2) : 307-313 (2007)) or a generic construct (see Courtete, Int. Advanced ICAS/APOTRAIN abstrackt book, abstract 15 (2008)) espressing sTRAIL.

The soluble TRAIL consists of the extracellular domain, vz. Amino acids 114-281 (see Shi, Cancer Res. 65(5): 1687-1692 (2005)) or 95-281 (see: Zhang, Acta Biochim. Pol 54(2) : 307-313 (2007)), fused with a signal peptideof human IgG (see : Zhang, Acta Biochim. Pol 54(2) : 307-313 (2007)).

Mohr, J. Cell.Mol. Med. 12(6b): 2628-2643(2008) discloses human mesenchymal stem cells expressing TRAIL from an adenovirus vector (as a fusion with E1, for treating tumors.

Nevertheless, this prior art still has certain drawbacks.

A first drawback is that the viral vector in use may have considerable restrictions of use due to its biological properties.

For instance, if an adenovirus is used, the greatest restriction consists in the inability of the viral genome to be stably integrated in the genome of infected cells, and this characteristic generates a transient, shortlasting form of TRAIL, which is designed to deplete, thereby causing a limitation of the duration of the therapeutic effect, like in the infusion of recombinant TRAIL.

An additional drawback, independent of the viral vector in use, is that the studies that have been conducted and published heretofore have concerned application of TRAIL only as a transmembrane protein and disregarded the existence of a biologically active domain of the molecule even in the form of a soluble ligand having a strong anti-tumor activity. This involved the generation of cell vectors that could only produce TRAIL cells as membrane proteins capable of inducing selective apoptosis of the target tumor cell only through direct contact allowed by interaction between the TRAIL on the carrier cell and its receptor on the target tumor cell; therefore, the membrane TRAIL carrying cell must be necessarily located in the proximity of or in contact with the tumor mass to ensure its therapeutic effect.

Another drawback is that the membrane TRAIL-carrying cell located in the tumor mass must survive and proliferate for the time required to exert its anti-tumor action; thus, the cell dose to be infused must be substantially comparable to the number of tumor cells to be eliminated, because cytotoxic action only occurs through cell contact.

As mentioned above, this causes high cell production costs and may give rise to side effects associated to infusion in the patient.

Another drawback is that the lack of an excreted TRAIL form has prevented more intensive pharmacokinetic studies on infused cells, and the duration of its effect with time could not be understood.

A further drawback is that carrier cells such as bone-marrow derived cells, have been used in the studies, without considering that these might produce molecules that can inhibit or even block the anti-tumor action of TRAIL molecules. The cell used as a carrier shall produce a small number of or no decoy receptors (such as OPGs), i.e. receptors capable of sequestering TRAIL, and preventing it from binding the receptor on the tumor cell.

Finally, the cell that is used as a carrier, in the case of stem cells, shall not have the TRAIL-specific receptors (DR4 and DR5) potentially capable of causing the suicide of the carrier cell, and hence hindering any therapeutic effect.

### Disclosure of the invention

The present invention improves on the prior art by providing an anti-tumor medicament that comprises adipose pericytes (AD-PC) stably expressing soluble TRAIL (sTRAIL) from a retroviral vector. Accordingly, the invention relates to:
1. A method for the production of a medicament for the treatment of a tumor, comprising:
   - preparing a retroviral vector encoding a soluble TRAIL molecule (sTRAIL), and
   - stably transfecting adipose pericytes (AD-PC) with said retroviral vector.
2. The method of [1], wherein the retroviral vector is produced irreversibly by a producer cell line.
3. The method of any one of [1] or [2]" wherein the retroviral vector is produced constantly and stably by a producer cell line.
4. The method any one of [1]-[3], wherein the AD-PC cell is an autologous, allogeneic, human or animal cell.
5. The method of any one of [1]-[4], wherein the soluble TRAIL is obtained by joining the TRAIL coding sequence to a sequence encoding a secretory peptide.
6. An anti-tumor medicament comprising an adipose perycite (AD-PC) cell stably transfected with a retrovirus expressing a soluble TRAIL molecule (sTRAIL).

### Brief description of the drawings

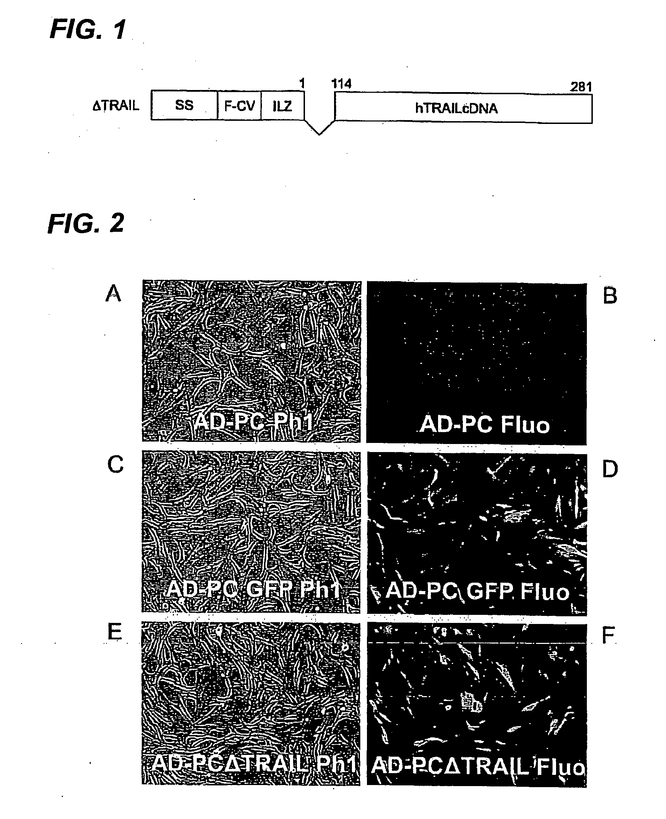

Figure 1 is a schematic representation of various functional domains of the ΔTRAIL construct, in which:
   - SS is a secretion signal or signal peptide;
   - F-CV (Furin-specific cleavage site) is a specific cleavage site for removal of the signal peptide by proteolytic cleavage after protein synthesis
   - ILZ (Isoleucine zipper) is a trimerization domain;
   - hTRAILcDNA is a sequence corresponding to human TRAIL from aa 114 to 281;
Figure 2 from A to F are reverse fluorescence microscope images (x 10 magnification) which show an optimal infection process given by the typically green color of the cells infected by a GFP-expressing viral vector (presence of the GFP protein) and in which:
   - A, B) cultured AD-PC with and without fluorescence respectively;
   - C, D) AD-PC GFP genetically modified by a retroviral vector encoding the GFP protein, with and without fluorescence respectively;
   - E, F) AD-PC ΔTRAIL with and without fluorescence respectively;
Figure 3 shows a phenotypic aspect of cultured AD-PC, AD-PC GFP e AD-PC ΔTRAIL, showing that handling of these cells with retroviral vectors (3B, C) does not change their aspect (X 10 magnification);
Figure 4 shows an immunophenotype, i.e. a cytofluorimetric analysis that shows how the retroviral infection did not alter the expression of AD-PC-characteristic phenotypic markers (CD45⁻, CD31⁻, CD146⁺);
Figures 5a to 5f show an expression of TRAIL receptors by cytofluorimetric analysis to define the receptor status (TRAIL-R1, TRAIL-R2) of TRAIL on AD-PC and on HeLa cells; particularly, Fig. 5a) shows the expression of TRAIL-R1 on AD-PC in a genetically unmodified condition; Fig. 5b) shows the expression of TRAIL-R2 on AD-PC in a genetically unmodified condition; Figs, 5c) and 5d) show the corresponding expressions of Figs. 5a) and 5b) in a TRAIL-producing genetically modified condition; Figs. 5e) and 5f) show the expressions of TRAIL R1 and R2 respectively, on HeLa tumor cells;
Figure 6 shows the an ELISA assay of OPG released in the supernatant of AD-PC and MSC (mesenchymal stem cells) from bone marrow, which shows that the OPG levels produced by AD-PC are much lower than the OPG levels released by bone marrow MSC;
Figure 7 is Western Blot of ΔTRAIL protein expression with columns 1 and 2 showing that the antibody has highlighted a band of about 21kDa corresponding to the ΔTRAIL protein in the protein lysate and in the supernatant of AD-PC ΔTRAIL whereas no band was detected at the AD-PC GFP protein lysate;
Figure 8 shows the result of a cytofluorimetric analysis on the expression of ΔTRAIL protein;
Figure 9 shows a quantitative ELISA assay of ΔTRAIL protein in AD-PC, AD-PC GFP and AD-PC ΔTRAIL supernatants: TRAIL values of 200-400 ng/ml are detected in AD-PC ΔTRAIL;
Figure 10 shows a quantitative ELISA assay of ΔTRAIL protein in sera of animals with AD-PC GFP and AD-PC ΔTRAIL 1x10⁵ and 1x10⁶: TRAIL values of 500-750 ng/ml are detected in AD-PC ΔTRAIL;
Figure 11 shows that AD-PC ΔTRAIL induces cell death in HeLa cells. The reversed microscope image (A) shows the presence of apoptotic bodies after 24 hours growth of HeLa cells in the supernatant of AD-PC ΔTRAIL (x 10 magnification). The cytofluorimetric analysis (B) shows a peak of cells displaced to high fluorescence values (Figure B higher) corresponding to an apoptotic event of HeLa in contact with the supernatant of AD-PC ΔTRAIL. No event was detected in HeLa cells in contact with the supernatant of AD-PC GFP;
Figure 12 shows caspase-8 activation. Culturing of HeLa cells with the supernatant of AD-PC ΔTRAIL induces caspase-8 activation in the tumor line. The figure shows that, after 8 hours contact with the supernatant of AD-PC ΔTRAIL, 40% of HeLa cells has caspase-8 activation (early apoptosis) and 50% is positive to 7-AAD, which identifies necrotic cells. Using a caspase-8 inhibitor, Z-VAD-FMK, the apoptotic process is stopped;
Figure 13 shows the weight of animals monitored during 60 days' treatment;.
Figures 14 and 15 show an assay of murine hepatic enzymes : no appreciable changes in AST and ALT concentrations have been found during 60 days' treatment;
Figure 16 shows the formation of a tumor mass monitored upon inoculation of AD-PC GFP and AD-PC ΔTRAIL. Control groups are found to show the appearance of a tumor mass after 20 days, unlike animals AD-PC ΔTRAIL-inoculated animals, in which tumor growth is strongly inhibited;
Figure 17 is a schematic representation of a cell division phase for an anti-tumor cell with a prior art adenovirus inserted therein;
Figure 18 is a schematic representation of a cell division phase for an anti-tumor cell with a retrovirus of the invention inserted therein,

### Detailed description of one non limiting, preferred embodiment

### EXAMPLE 1

- Isolation of pericytes from Vascular Stromal Fraction (VSF), immunophenotype characterization and gene modification: AD-PC cells were isolated from liposuctioned fat of healthy individuals through enzymatic digestion. The protocol includes processing of adipose tissue with a collagenase-based enzymatic solution. The buffer in which the lyophilized enzyme is resuspended is composed of the Low Glucose DMEM Medium (Dulbecco Modified Eagle's Medium, Euroclone) supplemented with 1% penicilline/streptomicine (PAA, Laboratories GmbH). Adipose tissue is incubated in the enzymatic solution at 37°C. Then, 1500 rpm centrifugation is applied for 10 minutes and the cell pellet is resuspended in Low Glucose DMEM supplemented with 10% FBS (Foetal Bocine Serum, PAA, laboratories GmbH) and 1% penicilline/streptomicine. The adipocytes left in the suspension are eliminated using a filter having 100 µm porosity (Cell Strainer, BD Falcon) and the filtrate is centrifuged at 1500 µm for 10 minutes. The pellet so obtained is resuspended and cells are counted by 0.4% trypan blue exclusion (Cambrex) (Cell counting in a Bürker chamber using a 10 X reverse microscope). Cells are later plated in a standard medium (PAA, Laboratories GmbH) supplemented with 1% penicilline/streptomicine and 1% L-Glutamine (PAA, Laboratories GmbH).

Immunophenotypic analysis was carried out for cultures at step 4. After trypsinization, cells were pelleted, resuspended in 100 µl blocking buffer and incubated for 30 minutes at 4°C. Then, the sample was incubated with the antibodies of interest, diluted in 90 µl PBS and 0.5% ABS (Albumin Bovine Serum, Sigma), for 20 minutes at 4°C.

The antigens whose expression was to be assessed were: CD45, CD31, CD146, TRAIL-R1, TRAIL-R2.

The cells so labeled were analyzed by a FACScalibur cytofluorimeter (Becton-Dickinson).

The ELISA assay allowed quantification of the OPG amount released by the cells of the supernatant.

The supernatant of AD-PC and bone marrow MSC was assayed according to the protocol for the Quantikine Human OPG/TNFRSF11B kit (R&D Systems, France) used for this assay.

Creation of the retroviral vector encoding solubile TRAIL (ΔTRAIL): the election strategy that was employed for cloning the SS-FurinCV-ILC-DTRAIL molecule in a retroviral vector was as follows: The total RNA isolated from peripheral blood mononuclear cells of a healthy donor was back-transcripted by means of SuperScript TM Reverse Transcriptase (Invitrogen, USA), using 2 µg total RNA as a template and random hexamers (Roche, Germany) as primers.

The encoding fragment for the extracytoplasmatic portion of TRAIL corresponding to amino acids 114-281 was obtained by PCR, from cDNA pool, with special primers: 5' -CAGATCTGGTGAGAGAAAGAGGTCCTCAGAGAGTA-3' (containing the cleavage site for BgIII) and 5'-GGAATTCCTTAGCCAACTAAAAAGGCCCC - 3' (including the cleavage site for EcoR1). By ligase reactions, the secretion signal, the specific cleavage site of the signal sequence (Furin-CV)and the trimerization domain (ILZ) were placed at teh 5' end of the gene sequence. The trimerization domain (ILZ) and the cleavage site obtained by PCR overlapping, were digested by the restriction enzymes XhoI-HindIII and HindIII-BgIII respectively. The two fragments were later cloned together with the extracytoplasmatic portion of TRAIL in a retroviral vector.

Creation of an AD-PC population stably expressing the soluble ΔTRAIL protein, and check of protein expression by Western Blot, cytofluorimetric analysis and ELISA assay: the creation of a retrovirus population capable of infecting the cell population of interest represented by AD-PC was articulated through two steps. The first step based on the obtainment of a cell line producing the retrovirus in transient mode and the second step aimed at obtaining the generation of a packaging cell line (PCL) capable of stably producing a retroviral progeny. For the transient step, embryonic renal fibroblasts (293 T cells), held at about 70% confluence were transfected with a solution of 5 µg (in a 25 cm² flask) total plasmid DNA and with the help of polycations. Later, the retroviral supernatant obtained by transient transfection of 293T was collected and used to infect the producer cell line (PLC) deriving from a human fibrosarcoma line; 24 h after infection the cells were analyzed by cytofluorimetry to check positivity of green fluorescent protein (GFP), an infection efficiency marker.

The retroviral supernatant collected by the stably ΔTRAIL-producing PCLs was collected and used to infect AD-PC cells. At least 3 infections, at 30 days from each other were performed with the same PCL, showing the stability of retroviral production.

AD-PC cells were seeded at a concentration of 5.7 x 10³/cm² 12 hours before infection, after replacement of the medium with a medium supplemented with a retroviral supernatant and 6µg/ml polybrene, then the cells were left for 6 hours in an incubator at 37°C, 5% CO2. The infection procedure was repeated for three days. Then, the cells were left in a standard medium for a few days before cytofluorimetric analysis to check GFP protein expression. ΔTRAIL protein production by infected AD-PC cells was assessed by Western Blot, cytofluorimetric analysis and ELISA assay.

Western Blot analysis allowed protein identification both in the cell lysate and the supernatant.

In short, the ΔTRAIL protein-expressing AD-PC cells were lysed in a lysis-buffer (50mM Tris-HCl, pH 7.4, 150mM NaCl, 1% NP-40, 1% Na-deoxycholate, 1mM EDTA, 0,1% SDS, Complete protease inhibitor). Protein concentration was determined by a Bio-Rad protein assay (Bio-Rad Laboratories, Italy), cell lysates and cell supernatants were run on 8% polyacrylamide gel.

Protein detection was allowed by incubation of the nitrocellulose filter with anti-h-TRAIL (K-18) goat polyclonal antibody (Santa Cruz Biotechnology, Inc., USA) as a primary antibody and anti-goat IgG-HRP (1:10000, Santa Cruz Biotechnology, Inc., USA) as a secondary antibody.

Cytofluorimetric analysis, that allowed detection of the protein in the cytoplasm of the cell, was carried out as follows: about 7 days after infection, the cells (500,000 cells/sample) were collected and permeabilized by Perm/Wash buffer (BD Biosciences, USA) and then were incubated with the PE conjugated anti-human TRAIL CD253 antibody (BioLegend, USA) and finally analyzed by a FACSCalibur cytofluorimeter (BD Biosciences, USA). Monoclonal PE conjugated mouse IgG1, kAs antibody (BD Biosciences, USA) was used as a control isotype.

The ELISA assay allowed quantification of the ΔTRAIL amount released by the cells of the supernatant.

The supernatant of ΔTRAIL-expressing AD-PC cells and the AD-PC infected by the empty vector was assayed according to the protocol for the Quantikine Human TRAIL/TNFSF10 kit (R&D Systems, France) used for this assay.

### RESULTS 1

In order to develop a retroviral vector encoding a soluble TRAIL form, the encoding region for the extracytoplasmatic portion of the molecule, having a proapoptotic biological activity, was fused with a sequence encoding a secretion signal (derived from the immunoglobulin heavy chain) and with a trimerization domain (Figure 1).

The soluble TRAIL solution so obtained was inserted in a retroviral vector also containing the GFP gene. A retroviral vector containing the GFP gene only was used as a control. These two vectors were used to transduce the pericytes isolated from adipose tissue in view of testing the actual anti-tumor activity of ΔTRAIL-expressing pericytes (AD-PC ^{Δ TRAIL}).

The success of the AD-PC infection process using a retroviral vector was assessed by reverse fluorescence microscopy (Figures 2A,2B,2C,2D,2E,2F). The optimal results of the infection process is confirmed by the presence of a wholly GFP-positive, fluorescent population (Figures 2D and 2F), whereas the uninfected control (Figure 2B) is negative when observed in the fluorescence channel.

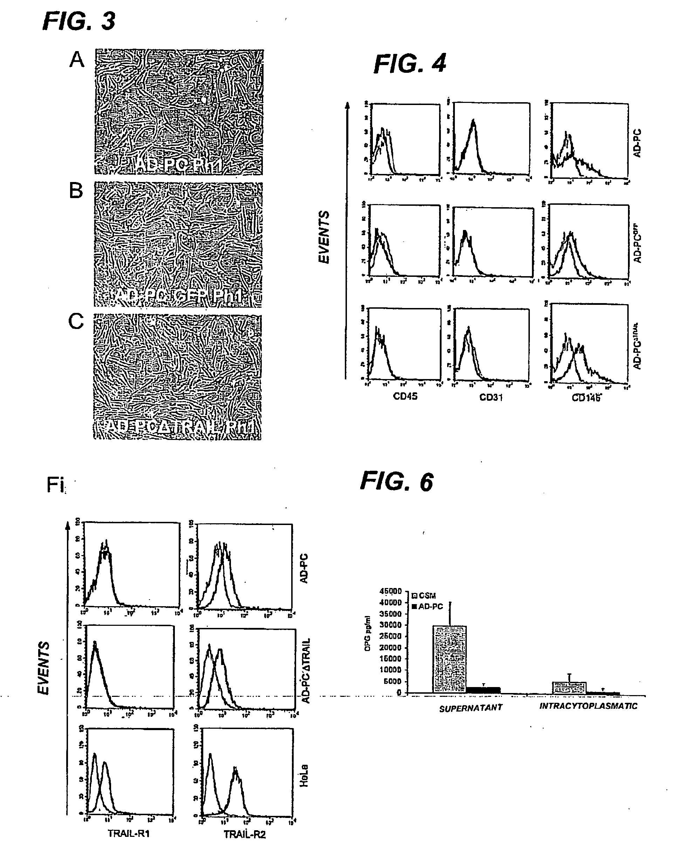

The AD-PC cells isolated from the Vascular Stromal Fraction (VSF) and observed in a microscope show a fibroblast morphology (Figure 3A), which is not subjected to changes upon genetic modification (Figures 3B and 3C). Then, the AD-PC cells were subjected to immunophenotypic assessment by cytofluorimetric analysis to check the expression of the typical markers of these cells before and after retroviral vector infection. It is noted that the immunophenotypic analysis of retroviral vector infected AD-PC cells (Figure 4, lower panel at the center) is identical to that of uninfected AD-PC cells (Figure 4, top panel), which indicates that the retroviral infection does not alter the expression of the characteristic phenotypic markers of the selected cell vector. Furthermore, the poor TRAIL-R1 and TRAIL-R2 expression, still shown by cytofluorimetric analysis, justifies the selection of AD-PC cells as cell vectors for carrying TRAIL (Figure 5a). Such reduced expression of these receptors, when compared with that shown by TRAIL-sensitive cells, such as HeLa (cervical cancer cell line), makes the AD-PC cells insensitive to apoptotic action exerted by trail and protects them from ligand-induced death.

Finally, still with the aim of evaluating the effectiveness of the selected cell carrier, the levels of OPG released in the supernatant by AD-PC cells were assayed by ELISA.

As frequently mentioned in the art, OPG acts as a "decoy" receptor which means that, since it is a soluble molecule having a good binding affinity for TRAIL, it can bind and sequester it thereby preventing any interaction thereof with TRAIL-R1 and TRAIL-R2 receptors and hence causing its biological activity to be blocked.

Therefore, the production of large amounts of OPG by the cell carried would prevent the use of such cell carrier in a TRAIL-mediated anti-tumor strategy, because most of the TRAIL produced by the cell would be readily sequestered by the OPG in the supernatant, and the anti-tumor effect would be consequently lost.

Nevertheless, as shown in Figure 6, the levels of OPG produced by AD-PC cells, both as a ligand soluble in the supernatant and as an intracytoplasmatic protein are irrelevant when compared with those produced by bone marrow mesenchymal stem cells MSC, that are mentioned in the art as carriers for TRAIL, which further confirms the novelty of the use of AD-PCs as a cell carrier.

The transduced AD-PC cells were assayed to check actual TRAIL expression by cytofluorimetric analysis and Western Blot.

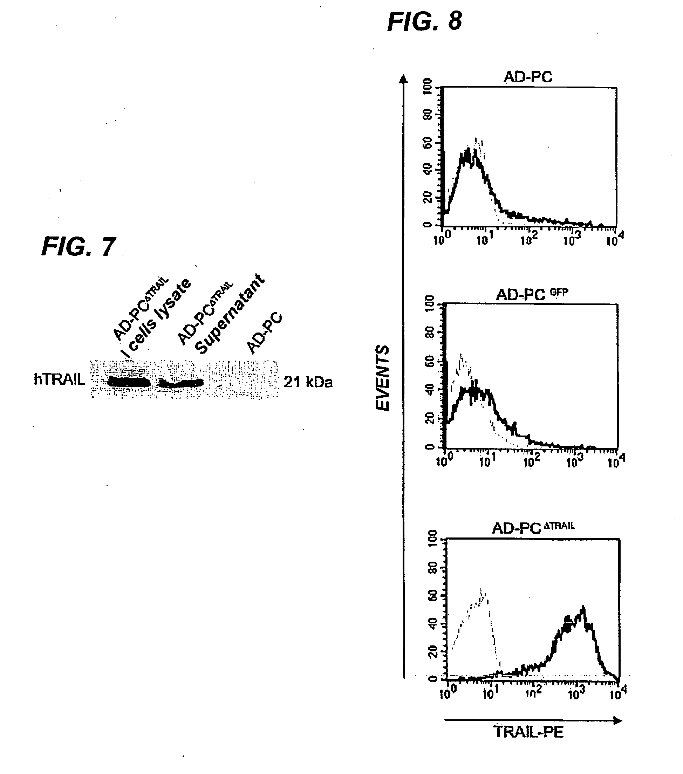

When looking at the Western Blot image (Figure 7) the TRAIL protein band can be identified both in the cell lysate of AD-PC^{ΔTRAIL}, confirming the presence of the protein at intracytoplasmatic level, and in the supernatant, confirming that, once the protein has been produced by the cell, it is excreted into and extracytoplasmatic environment. This is particularly relevant when considering that, for the suggested application, the cell would be infused in the organism, whereby TRAIL excretion in an extracytoplasmatic environment would cause release thereof in the blood flow, thereby leading to a systemic anti-tumor effect independent of the position of the cell carrier relative to the tumor mass.

Protein production by AD-PC^{ΔTRAIL} cells was also validated by submitting the cells to cytofluorimetric analysis (Figure 8), which allowed identification of TRAIL at intracytoplasmatic level thereby confirming Western Blot analysis.

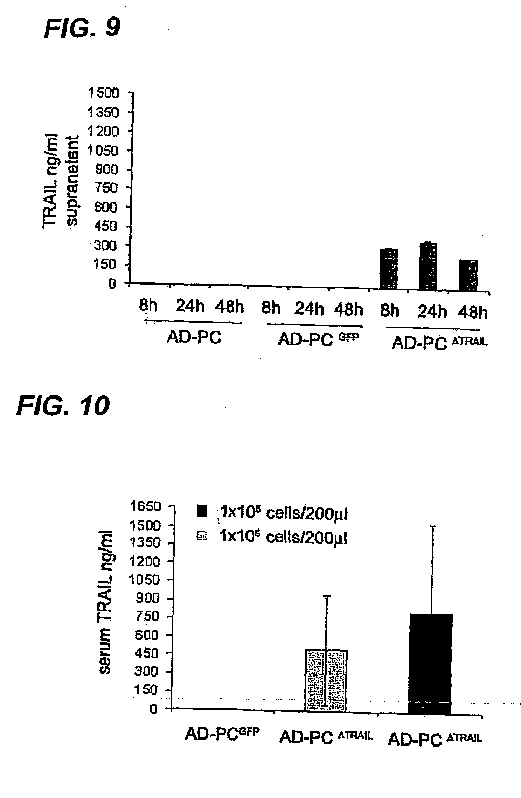

The next step after protein identification was quantification by ELISA assay. TRAIL values released in culture media by AD-PC, AD-PC^{GFP} and AD-PC^{ΔTRAIL} cells were assayed at 8, 24, 48 hours' growth. The detected values (200-400ng/ml) are not susceptible of relevant changes at the different time intervals. This may be indicative of the fast protein turnover: the amount of degraded protein corresponds to that of produced protein (Figure 9).

### EXAMPLE 2

- Evaluation of the cytoxic effect of ΔTRAIL on tumor cells: HeLa tumor cells were seeded in 24 well plates at a concentration of 6x10³/well in triplicate 24 hours before the assay.

The supernatant, that was left in contact with AD-PC^{ΔTRAIL} or AD-PC^{GFP} cells for 4, 24 and 48 hours was collected to replace the medium in the wells in which the tumor cells had been seeded. Cell viability and necrosis were assessed after 4, 24 and 48 hours by propidium iodide staining (50µg/ml) followed by cytofluorimetric analysis. As a positive control, the same tumor cells, under the same conditions, were placed in contact with 20 ng/ml recombinant soluble human TRAIL protein (Peprotech, Rocky Hill, NJ).

Caspase-8 activation assay: the activation of caspase-8, an enzyme involves in TRAIL-mediated early pro-apoptotic pathway is assessed as follows: HeLa tumor cells are put in contact with the supernatant collected from AD-PC ^{ΔTRAIL}. A negative control is also prepared, which consists of the same sample with the addition of a caspase inhibitor (Z-VAD-FMK 10µM). After 4 and 8 hours treatment, the cells are trypsinized, resuspended in 300 µl PBS and incubated for 45 minutes at 37° in the dark with RED-IETD-FMK (sulforhodamine conjugate). The bond of this molecule to caspase-8, activated in apoptopic cells is detected by fluorescence using a cytofluorimeter.

### RESULTS 2.

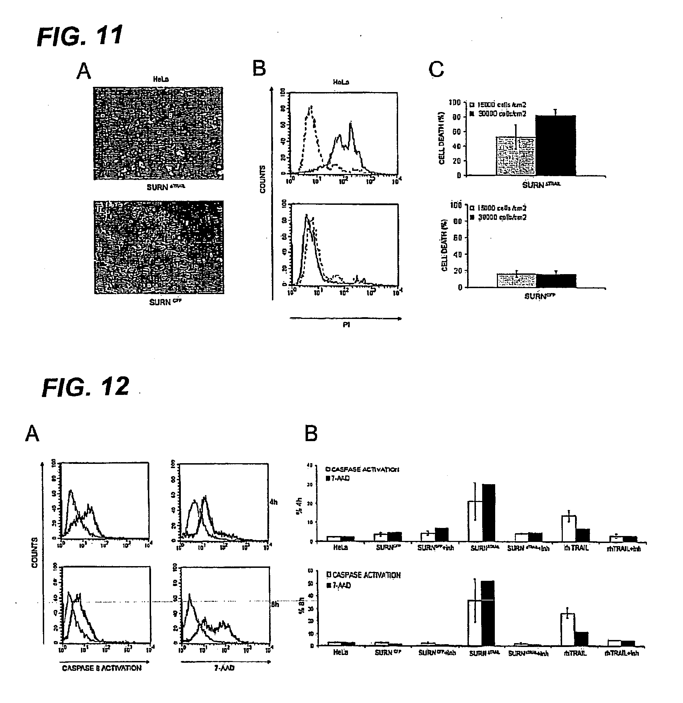

When looking at the reverse microscopy images that show the morphology of HeLa cells when in contact with the supernatant of AD-PC^{ΔTRAIL} cells (Figure 11A, higher) as compared with that of HeLa in contact with the supernatant of AD-PC^{GFP} cells (Figure 11A, lower) cell distress is found in the former case, which is characterized by the presence of a large number of apoptotic bodies and debris fluctuating in the medium. The mortality of HeLa placed in contact with the supernatant of AD-PC^{ΔTRAIL} cells was quantified by cytofluorimetric analysis using propidium iodide (PI). PI is a non vital stain intercalating in the DNA of necrotic and advanced apoptotic cells.

Cytofluorimetric representation of 24 hours mortality of Hela in contact with the supernatant of AD-PC^{ΔTRAIL} cells as shown in Figure 11B (higher) where the peak of positive PI cells is found to be displaced toward high fluorescence values as compared with the corresponding peak of HeLa treated with a supernatant of AD-PC^{GFP} cells (Figure 11B lower) and to the peak of overlay-plaqued HeLa (broken line).

A more intensive analysis confirms this data: treated HeLa cells at 4, 24 and 48 hours with the supernatant deriving from hyperconfluent AD-PC^{ΔTRAIL} (30,000cellule/cm²) exhibit a mortality of more than 80%, whereas the mortality of HeLa placed in contact with the conditioned medium of AD-PC^{GFP} (30,000 cells/cm²) is not more than 20% (Figure 11). Then, considering the mortality percentages of HeLa left in contact with the supernatant of lower confluence AD-PC^{ΔTRAIL} cells(15,000 cells/cm²), a mortality drop will apparently occur. This result indicates the existence of a correlation between the amount of AD-PC^{ΔTRAIL} cells and the concentration of TRAIL released in the medium, as previously shown by the ELISA assay. In order to show that the mortality detected on HeLa is really induced by the anti-tumor action mediated by ΔTRAIL excreted by infected AD-PC cells, the cleavage of caspase-8, which represents the first mediator involved in the translation of the TRAIL-mediated pro-apoptotic signal was observed in tumor cells.

Cytofluorimetric analysis showed that, after 8 hours, 40% of the tumor cells treated with the supernatant of AD-PC^{ΔTRAIL} cells showed cleavage of caspase-8, which event represents an early step in the apoptotic process of the cell, and at the same time staining with 7AAD (analog of propidium, late death stain) shows 50% of necrotic cells, these values are definitely higher than those obtained with recombinant TRAIL (20 ng) which correspond to 8 h at 25% for caspase-8 activation and 10% for 7AAD positivity (Figure 12). Finally, concerning the controls treated with the supernatant of AD-PC^{GFP} cells, caspase-8 activation and 7AAD positivity reach values comparable to the untreated control. The presence of the caspase-8 inhibitor (Z-VAD-FMK) in the ΔTRAIL containing supernatants brings the percentage of positive cells to values comparable to the control of untreated cells or cells treated with the supernatant derived from AD-PC^{GFP} cells, and a similar situation is observed 7AAD positivity is evaluated in the presence of the inhibitor, the death of cells is reduced in the presence of Z-VAD-FMK. An identical behavior, although with slightly lower percentage values, is obtained for the 4 treatment hours.

This data both confirms that the mortality encountered with treated tumor cells is really mediated by the anti-tumor action exerted by the ΔTRAIL excreted by infected AD-PC cells, and reinforce the data obtained by analysis with PI, which showed, at 12 hours from treatment, 80% mortality correlating with 40% of early apoptosis cells (caspase-8 positive) and with 50% of late apoptosis cells (7AAD positive) encountered at 8 hours (Figure 12B).

### EXAMPLE 3

- Assessment of the *in vivo* anti-tumor effect of AD-PC ^{Δ TRAIL} cells: for *in vivo* studies, eight-ten week NOD. CB17-Prkdc ^{scid}/J mice were used, according to the guidelines of experimental protocols (Prot./n.543) approved by the ethic committee on animal experimentation of the University of Modena and Reggio Emilia. 2 x 10⁵ HeLa in 200 µl di PBS were subcutaneously administered to the animals. The tumor disappeared about two weeks later, its size being constantly monitored by means of a tumorimeter. Tumor volume was calculated using the formula: volume = length x height²/2.

As the tumor appeared, the mice were divided into three groups, each consisting of three mice, the animals of the group A (control group) underwent additional handling or even weekly evaluation of tumor mass and weight growth, the animals of group B underwent three successive administration hits, by injection of 1 x 10⁶ AD-PC^{GFP} resuspended in 200 µl PBS into the lateral tail vein, the animals of group C underwent three successive administration hits by injection of 1 x 10⁶ AD-PC^{ΔTRAIL} resuspended in 200 µl PBS 9 ino the lateral tail vein.

At the end of the test (60 days), the animals were sacrificed and the tumor was excised.

In order to assess the presence of AD-PC ^{ΔTRAIL} cells in tissues other than the tumor site, biopsies were performed on the spleen, liver, kidney, heart, muscle, lung, skin femur from which the DNA was extracted.

Research of AD-PC^{GFP} and AD-PC^{ΔTRAIL} cells in mouse healthy tissues: extraction of DNA from murine tissue was carried out using the Isolation kit (Gentra Systems). In order to detect the presence of GFP from isolated tissues, the extracted DNA was amplified using GFP-specific primers, 5' -GTAAACGGCCACAAGTTCAG-3' and 5'-TGTCGGCCATGATATAGACG -3' (PubMed DQ768212) (fragment 402bp). The amplificability and integrity of the template have been checked by amplification of the GAPDH constitutive gene using specific primers 5'-GCAGTGGCAAAGTGGAGATT -3'; 5'-GCAGAAGGGGCGGAGATGAT -3', 308BP (PubMed XM_973383).

### RESULTS 3

The animals were regularly monitored during testing to assess their physical conditions.

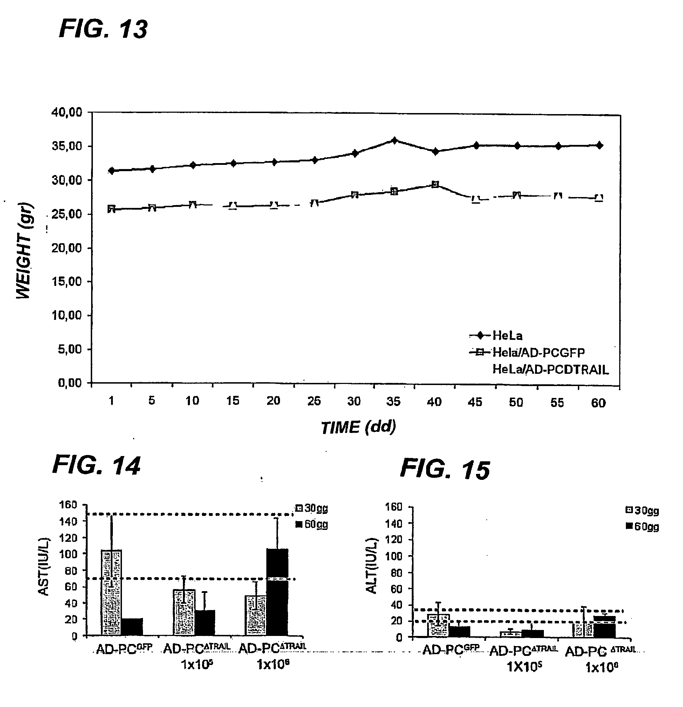

A weight analysis was weekly performed on mice receiving AD-PC, AD-PC^{GFP} and AD-PC^{ΔTRAIL} inoculations and no considerable weight change has been noted (Figure 13). Hepatic toxicity was found in the art in association with TRAIL administration, therefore careful monitoring was carried out on the activity of hepatic enzymes of inoculated animals, which did not show hepatic distress (Figures 14-15). This is very important, because the presence of ΔTRAIL expressing cells at systemic level (Figure 10) in the animal was tested by assaying the serum of animals by ELISA to find the TRAIL protein. In animals inoculated with AD-PC 1 x 10⁵, the detected TRAIL levels are lower than those inoculated with AD-PC 1 x 10⁶ at sixty days treatment. This data provides an accurate indication on the permanence of AD-PC cells in the animals after several days from inoculation and also on constant TRAIL production thereby.

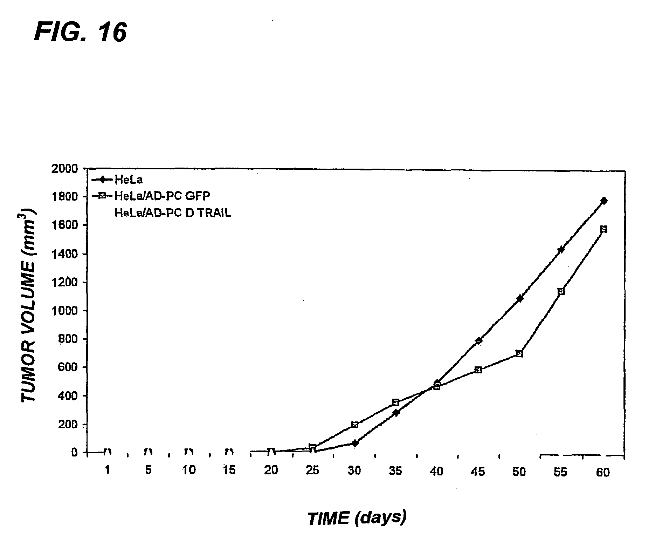

Systemic administration of AD-PC^{□TRAIL} cells after formation of the tumor mass (HeLa), allowed testing of both the migrating ability of said cells and their tumor growth inhibiting action, Figure 16 shows the behavior of the tumor neoformation upon inoculation of AD-PC^{GFP} and AD-PC^{ΔTRAIL}. The control groups show an ascending behavior of the tumor mass growth from the fifteenth-twentieth day from inoculation, whereas the group of AD-PC^{ΔTRAIL} inoculated animals shows a quasi basal behavior of tumor formation, which can be identified by an inhibition of tumor growth.

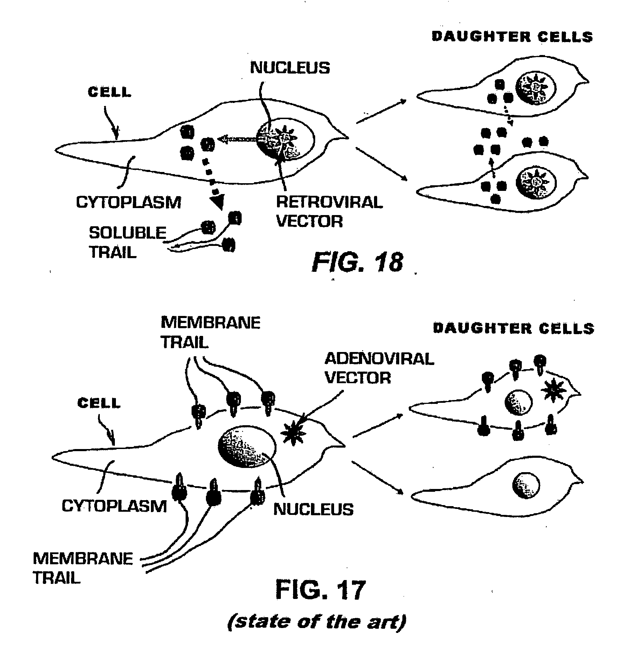

Referring to Figure 17, it will be appreciated that, in prior art, a membrane TRAIL-producing anti-tumor cell, whose nucleus contains an adenovirus that remains in episomal form (not integrated in the genome of said cells), produces two daughter cells when it is divided, one of which maintains the adenovirus that can produce membrane TRAIL, whereas the other is completely free of it; this causes a TRAIL production dilution effect at every cell division.

However, it is noted in Figure 18 that, according to the invention, an anti-tumor cell with a retrovirus integrated in its nucleus, generating soluble TRAIL when it is divided, produces two identical daughter cells that both maintain the retrovirus integrated in their nuclei; therefore, both can produce soluble TRAIL, thereby eliminating the effect of dilution observed in prior art.

## Claims

1. A method for production of a medicament for the treatment of tumor comprising: preparing a retroviral vector encoding a soluble TRAIL molecule (sTRAIL), and stably transfecting adipose pericytes (AD-PC) with said retroviral vector.

2. The method according to claim 1, wherein said retroviral vector is produced irreversibly by a producer cell line.

3. The method of anyone of claims 1 or 2, wherein the retroviral vector is produced constantly and stably by a producer cell line.

4. The method of anyone of claims 1-3, wherein the (AD-PC) cells is an autologous, allogenic, human or animal cell.

5. The method of anyone of claims 1-4, wherein the soluble TRAIL is obtained by joining the TRAIL coding sequence to a sequence encoding a secretory peptide.

6. An anti-tumor medicament comprising an adipose perycite (AD-PC) cell stably transfected with a retrovirus expressing a soluble TRAIL molecule (sTRAIL).

## Patentansprüche

1. Ein Verfahren zur Erzeugung eines Medikaments zur Tumorbehandlung mit den Schritten:
Zubereiten eines retroviralen Vektors, der für ein lösliches TRAIL Molekül codiert und stabiles Transfizieren von adipösen Pericyten (AD-PC) mit diesem retroviralen Vektor.

2. Verfahren gemäß Anspruch 1, **dadurch gekennzeichnet, dass** dieser retrovirale Vektor irreversibel erzeugt wird mittels einer Erzeuger-Zellreihe.

3. Verfahren gemäß einem der Ansprüche 1 oder 2, **dadurch gekennzeichnet, dass** der retrovirale Vektor konstant und stabil erzeugt wird mittels einer Erzeuger-Zellreihe.

4. Verfahren gemäß einem der Ansprüche 1-3, **dadurch gekennzeichnet, dass** die (AD-PC) Zellen eine autogenetische, allogenische, menschliche oder tierische Zelle ist.

5. Verfahren gemäß einem der Ansprüche 1-4, **dadurch gekennzeichnet, dass** der lösliche TRAIL erhalten wird mittels Verbinden der TRAIL kodierenden Sequenz mit einer Sequenz, die für ein sekretorisches Peptid kodiert.

6. Ein Anti-Tumormedikament mit einer adipösen Pericyt (AD-PC) Zelle, die stabil transfiziert ist mit einem Retrovirus, der für ein lösliches TRAIL Molekül (sTRAIL) exprimiert.

## Revendications

1. Méthode pour produire un médicament pour traiter les tumeurs, comprenant : un vecteur rétroviral codant une molécule TRAIL soluble (sTRAIL), et transfectant stablement des péricytes adipeux (AD-PC) avec ledit vecteur rétroviral.

2. Méthode selon la revendication 1, où ledit vecteur rétroviral est irréversiblement produit par une lignée cellulaire productrice.

3. Méthode selon l'une quelconque des revendications 1 ou 2, où le vecteur rétroviral est constamment et stablement produit par une lignée cellulaire productrice.

4. Méthode selon l'une quelconque des revendications 1-3, où les cellules (AD-PC) sont une cellule humaine ou animale, autologue, allogénique.

5. Méthode selon l'une quelconque des revendications 1-4, où le TRAIL soluble est obtenu en associant la séquence de codage TRAIL à une séquence codant un peptide sécrétoire.

6. Médicament anti-tumeur comprenant une cellule péricyte adipeuse (AD-PC) stablement transfectée avec un rétrovirus formulant une molécule TRAIL soluble (sTRAIL).
